# DEMANDE DE BREVET EUROPEEN

(11) **EP 2 874 085 A1**
(43) Date de publication de la demande: **20.05.2015**
(21) Numéro de dépôt: 14193738.3
(22) Date de dépôt: 18.11.2014
(51) Int. Cl.: G06F 19/18, G06F 19/22

(54) **Procédé et dispositif pour le traitement reparti de données génomiques**

(30) Priorité: 18.11.2013 FR 1361323
(71) Demandeur: Dominguez Del Angel, Victoria, 91300 Massy (FR)
(72) Inventeur: Dominguez, Victoria, 91300 Massy (FR); Chebbi, Amine Mohamed, 75015 Paris (FR); Bortzmeyer, Stéphane, 75015 Paris (FR); Fanny, Michel, 75017 Paris (FR)
(74) Mandataire: Lecomte & Partners

(57) **Abrégé**

L'invention concerne la bio-informatique et propose un procédé de traitement informatique réparti de données génomiques ou transcriptomiques. Le procédé est remarquable en ce qu'il permet le traitement rapide, traçable et reproductible de volumes de données génomiques ou transcriptomiques importants. Le traitement est réalisé de manière distribuée par un ensemble de moyens de traitement, coordonnés par au moins une entité centrale. L'invention propose également un programme d'ordinateur mettant en oeuvre le procédé selon l'invention, comme un dispositif configuré pour la mise en oeuvre du procédé.

## Description

### Domaine de l'invention

La présente invention concerne la bio-informatique, et plus particulièrement des procédés, des systèmes, des méthodes pour le traitement et l'analyse réparti des données génomiques et/ou transcriptomiques.

### Contexte de l'invention

L'essor de la génomique a été permis, au cours des dernières années, grâce au développement et à la robotisation des technologies de séquençage nucléotidique de nouvelle génération (NGS) ou technologies de séquençage de seconde, voire de troisième génération. Ces technologies permettent de générer rapidement des quantités massives de données de séquences à faible coût. Elles ont ouvert la porte à un champ remarquable d'applications, à la fois pour les espèces modèles et non modèles. Une des plus vastes applications des NGS est le reséquençage des génomes pour lesquels un génome de référence est déjà disponible. Le reséquençage de génome permet d'identifier les variations génétiques individuelles afin de comprendre les bases génétiques des différences phénotypiques. Ainsi, des projets visant à séquencer le génome de nombreux individus au sein d'une même espèce, telle que l'homme (1000 Genomes Project Consortium) ou d'autres organismes tels que par exemple Arabidopsis thalian, ont émergé.

La transcriptomique a aussi permis une avancée remarquable. Le transcriptome représente l'ensemble des ARN issus de l'expression du génome (séquences transcrites) dans un type cellulaire donné à un moment donné et dans une condition biologique précise. L'un des principaux mécanismes initiant les processus adaptatifs dans une cellule est la modification des niveaux de transcription des gènes qui conduit à des changements dans la machinerie cellulaire. L'objectif principal de l'étude du transcriptome consiste donc à quantifier les niveaux d'expression des gènes pour un stade de développement ou une condition physiologique donnée. La transcriptomique permet ainsi, d'avoir un aperçu du fonctionnement interne d'un génome dans un tissu ou une condition physiologique donnée. Cette discipline est donc, fondamentale pour la compréhension des mécanismes gouvernant les processus biologiques et plus précisément de l'ARN. Ainsi, des banques d'ADN complémentaire (ADNc) sont synthétisées à partir des ARNm extraits d'un échantillon de cellules ou de tissus d'un organisme. Ces séquences d'ADNc, appelées étiquettes de séquences exprimées (ESTs), ne représentent que des fragments des transcrits d'origine, compte tenu de la dégradation rapide de certains ARNm. Le raisonnement qui sous-tend cette approche est que la représentation des séquences issues du séquençage profond des ADNc est proportionnelle à la quantité d'ARN du gène dans l'échantillon initial. L'abondance de chaque transcrit au sein des différents échantillons peut ensuite être comparée.

Les analyses des données génomiques et/ou transcriptomiques sont cruciales dans le domaine de la biologie et de la biologie moléculaire pour découvrir de nouveaux marqueurs de diagnostic, de nouvelles molécules cibles et des approches thérapeutiques par exemple dans la pharmacogénomique. Ces analyses sont également cruciales dans le domaine de la génomique pour l'analyse de l'expression des gènes, l'épissage alternatif, pour la découverte de nouveaux ARN (RNA editing), d'expression spécifique d'allèles, et l'exploration des transcriptomes dans les organismes non modèles.

Ces données permettent d'identifier les variations génétiques ou d'expression des gènes qui sont une composante majeure, que ce soit comme cause ou comme effet, de maladies, comme le cancer, le diabète les maladies cardiovasculaires, et les maladies neurodégénératives. Les risques de maladie peuvent être identifiés par des variations des gènes responsables. Cela peut être fait en analysant les mutations d'une base nucléotidique unique, appelé polymorphisme nucléotidique (SNP). Malheureusement, même si les SNPs peuvent indiquer quel médicament sera le mieux pour un individu donné, l'analyse SNP devra peut-être nécessiter des études humaines à grande échelle pour établir ces associations utiles. Sans analyse automatisée et pertinente, cela peut rendre le processus d'analyse des SNP processus coûteux et difficile.

En effet, la stratification des patients donnent un avantage clinique et permettent d'orienter la sélection des médicaments vers les patients qui présentent la plus forte probabilité de réagir positivement à certains médicaments.

Au cours de ces dernières années, l'explosion et la complexité des données génomiques et/ou transcriptomiques dues en particulier, aux développements importants des technologies de séquençage de nouvelle génération (NGS), ont entraîné inévitablement un accroissement de l'écart entre les données brutes générées par les séquenceurs, et les connaissances et les informations biologiques que l'on peut en extraire. Les progrès des technologies de séquençage ont été tout à fait spectaculaires. On parle dans les horizons, de nouvelles technologies de séquençage de masse. Les technologies NGS s'enchainent avec des coûts de plus en plus abordables, produisant des séquences plus longues et volumineuses. Cette accélération de séquençage introduira certainement, une grande révolution dans l'ère de la recherche génomique et médicale. On prévoit en France, qu'au bout des dix prochaines années, chaque hôpital sera équipé d'au moins un séquenceur de nouvelle génération dans le cadre du développement de la médecine translationnelle.

L'analyse et l'interprétation des données par l'outil informatique deviennent ainsi l'étape cruciale. Cette production toujours plus importante de données à analyser nécessite un accroissement des besoins de calcul et de stockage, et des outils informatiques avec des capacités de calcul et de stockage toujours plus importantes.

Il est donc important de mettre en place des procédés et des dispositifs pour le traitement et l'analyse des données génomiques et/ou transcriptomiques, le stockage des analyses des données génomiques et/ou transcriptomiques et de leurs mises à jour régulières, ainsi que pour la sécurisation des résultats de ces analyses.

Plusieurs méthodes relevant de la statistique et de l'algorithmique sont en développement permanent afin d'élaborer des stratégies d'analyses assez pertinentes. Mais ceci, reste insuffisant surtout en ce qui concerne les traitements et les analyses complexes faisant appel à une pluralité de logiciels qu'il faut chaîner les uns aux autres afin d'obtenir et reproduire les résultats désirés.

La présente invention a pour objectif de palier à au moins un de problèmes présents dans l'état de la technique, en proposant un procédé et des dispositifs qui permettent le traitement distribué ou réparti de données génomiques et/ou transcriptomiques de manière dynamique et reproductible.

### Résumé de l'invention

L'invention a pour objet un procédé de traitement informatique réparti ou distribué de données génomiques ou transcriptomiques. Le procédé comporte les étapes suivantes réalisées par des moyens de traitement centralisés:
- mise à disposition de données génomiques destinées à être traitées à l'aide de moyens de lecture faisant partie des moyens de traitement centralisés;
- détermination d'étapes de traitement nécessaires, workflow, pour réaliser ledit traitement;
- calcul d'une mesure indicative du niveau de complexité des données génomiques ou transcriptomiques;
- détermination de moyens de traitement répartis et de ressources de calcul allouées aux moyens de traitement répartis, en dépendance des étapes de traitement déterminées et de la mesure de complexité calculée, chacun des moyens de traitement répartis étant destiné à réaliser au moins une des étapes de traitement ;
- transmission d'au moins une partie des données génomiques et des ressources de calculs allouées à chacun des moyens de traitement répartis;
- réception des résultats de traitement générés par les moyens de traitement répartis.

Da manière préférée, les résultats reçus peuvent être combinés pour être stockés par les moyens de traitement centralisés. Les résultats peuvent également être présentés de façon textuelle et/ou graphique sur un dispositif d'affichage relié de manière opération elle aux moyens de traitement centralisés.

De manière préférée, les étapes de traitement peuvent être sélectionnées par les moyens de traitement centralisés en fonction du traitement à réaliser parmi un ensemble d'étapes de traitement prédéterminé, stocké dans une mémoire accessible aux moyens de traitement centralisés. La mémoire peut de préférence être structurée sous forme d'une base de données mettant en relation un type de traitement avec un ensemble d'étapes constituant un workflow. L'élément de mémoire qui supporte la base de données peut être accessible à distance à l'aide d'un réseau de communication par les moyens de traitement centralisés, ou être physiquement relié au dispositif informatique dont les moyens de traitement centralisés font partie.

Les ressources de calcul allouées aux moyens de traitement répartis peuvent de préférence être sélectionnées par les moyens de traitement centralisés parmi un ensemble de ressources de calcul prédéterminé, stocké dans une mémoire accessible aux moyens de traitement centralisés. L'élément de mémoire peut par exemple être une base de données.

De préférence, les ressources de calcul allouées à chacun des moyens de traitement répartis peuvent comprendre une quantité de mémoire, une quantité de cycles de calcul ou une quantité de largeur de bande de disque pour être utilisée pendant la réalisation de l'étape de traitement associée aux moyens de traitement répartis.

La mesure indicative du niveau de complexité des données génomiques ou transcriptomiques peut préférentiellement être calculée à l'aide d'une ou de plusieurs fonctions de complexité choisies parmi :
(i) la fonction Wootton & Federhen, FCF ;
(ii) la fonction de l'entropie de complexité, CE ;
(iii) la valeur des modèles de Markov, CM ;
(iv) les valeurs linguistiques de complexité, CL ;
(v) la complexité Zlib, ZL ;
(vi) les valeurs Trifonov, CT ;
(vii) une normalisation de ces fonctions.

Le procédé peut de manière préférentielle comprendre en outre une étape de stockage des données génomiques, des étapes de traitement déterminées, des ressources de calcul allouées aux moyens de traitement répartis réalisant les étapes de traitement, ainsi que des résultats générés par lesdits moyens de traitement distribués. Le stockage permet donc d'enregistrer tout le datapath et de permettre à des tiers la reproductibilité de toutes les étapes de traitement de données effectuées. La traçabilité des résultats obtenus peut ainsi être garantie.

Les données stockées peuvent de préférence être écrites par les moyens de traitement centralisés dans une base de données à laquelle les moyens de traitement peuvent accéder. La base de données peut être accessible à l'aide d'un réseau de communication ou l'élément de mémoire qui la supporte peut être physiquement relié au dispositif dont les moyens de traitement centralisés font partie.

De préférence, chacun des moyens de traitement répartis peut être configuré de façon à stocker dans un élément de mémoire les données génomiques reçues, l'allocation de ressources de calcul reçue et les étapes de traitement allouées par les moyens de traitement, ainsi que les résultats des étapes de traitement générés. Le stockage permet à des tiers la reproductibilité de toutes les étapes de traitement de données effectuées.

Les moyens de traitement répartis peuvent de préférence être des moyens de traitement d'un dispositif informatique unique. Avantageusement les moyens de traitement répartis peuvent être des machines virtuelles utilisant les ressources physiques d'un ou de plusieurs dispositifs informatiques.

Alternativement, les moyens de traitement répartis peuvent être des moyens de traitement de différents noeuds dans un réseau de communication. Encore alternativement, les moyens de traitement répartis peuvent être en partie des machines virtuelles et en partie des dispositifs physiques dédiés.

De préférence, l'étape de transmission peut être réalisée à l'aide de moyens de transmission de données sécurisés.

L'invention a également pour objet un programme d'ordinateur comprenant des moyens de code lisibles par ordinateur, qui, lorsqu'il est exécuté sur un ordinateur, amène l'ordinateur à exécuter le procédé selon l'invention.

L'invention a également pour objet un produit de programme informatique comprenant un support lisible par ordinateur sur lequel le programme d'ordinateur selon l'invention est stocké.

De même, l'invention a pour objet un ordinateur capable de mettre en oeuvre le procédé selon l'invention

Finalement, l'invention a également pour objet un dispositif pour la mise en oeuvre du procédé selon l'invention. Le dispositif comprend des moyens de lecture, des moyens de communication pour transmettre des données vers et pour et recevoir des données de moyens de traitement répartis, un élément de mémoire et des moyens de traitement. Les moyens de traitement sont configurés de façon à :
- lire des données génomiques ou transcriptomiques destinées à être traitées à l'aide des moyens de lecture;
- déterminer un ensemble d'étapes de traitement nécessaires pour réaliser ledit traitement;
- calculer une mesure indicative du niveau de complexité des données génomiques;
- déterminer un ensemble de moyens de traitement répartis et de ressources de calcul allouées aux moyens de traitement répartis, en dépendance des étapes de traitement déterminées et de la mesure de complexité calculée, chacun des moyens de traitement répartis étant destiné à réaliser au moins une des étapes de traitement ;
- transmettre au moins une partie des données génomiques et des ressources de calculs allouées à chacun des moyens de traitement répartis;
- recevoir des résultats de traitement générés par les moyens de traitement répartis.

De préférence, le dispositif comprend des moyens d'accès en écriture et/ou en lecture à une ou plusieurs bases de données.

Le procédé selon la présente invention permet la structuration du flux de travail, workflow, et la transmission de données nécessaires à l'analyse génomique ou transcriptomique. Le procédé est mis en oeuvre par un ensemble de machines (virtuelles ou physiques) interconnectées. Cet ensemble comprend une ou plusieurs machines spécialisées ou machines esclaves ayant pour tâche d'exécuter de manière distribuée une ou plusieurs analyses statistiques des données génomiques et/ou du stockage des résultats. L'ensemble comprend également et au moins une machine maître ayant pour tâche de définir les étapes de traitement à réaliser, de transmettre le flux de données à une ou plusieurs machines spécialisées ou machines esclaves pour exécution des analyses et du stockage du datapath complet utilisé pour obtenir les résultats d'analyse.

Les étapes de traitement peuvent notamment comprendre de manière non limitative des étapes de détermination de la nature des données génomiques brutes, des étapes de filtrage de ces données, des étapes de conversion de données.

Un ensemble de dispositifs implémentant au moins une machine maître (ou des moyens de traitement centralisés) et plusieurs machines esclaves (ou moyens de traitement répartis/distribués) interconnectés de manière opérationnelle a pour tâche d'exécuter une ou plusieurs analyses des données génomiques et statistiques et optionnellement le stockage des résultats.

L'invention permet de procéder à une analyse automatique de données génomiques de nature variée. Le procédé et les dispositifs selon la présente invention permettent de surmonter les problèmes techniques décrits et de gérer des quantités de données importantes ainsi générées. L'utilisation de moyens de traitement distribués permet de réaliser au moins une partie des tâches en parallèle, ce qui engendre une diminution importante du temps de traitement effectif.

Le stockage des configurations utilisées pour réaliser chaque étape du traitement, datapath, avec les données non traitées et traitées, permettent la traçabilité et la reproductibilité d'exécution des étapes qui ont permis d'obtenir ces résultats. Notamment, une analyse postérieure de ces données permet le cas échéant d'adapter ou d'optimiser les ressources de calcul allouées à chacun des moyens de traitement répartis en fonction des étapes de traitement à réaliser. Les nouveaux résultats sont immédiatement disponibles pour de prochains traitements. Le principe modulaire du procédé selon l'invention permet également d'intégrer de nouveaux outils d'analyse et/ou de traitement dans un workflow de traitement, sans pour autant changer le procédé selon l'invention. Il suffit de mettre à disposition les étapes de traitement correspondants et les outils correspondants dans l'ensemble des étapes de traitement prédéterminé.

La configuration des moyens de traitement répartis est donc modulable et configurable en fonction de nouveaux résultats obtenus.

### Brève description des figures

D'autres caractéristiques et avantages de la présente invention seront mieux compris à l'aide de la description exemplaire et des dessins parmi lesquels :
- la figure 1 est une illustration schématique des étapes principales du procédé selon l'invention dans un mode de réalisation préféré ;
- la figure 2 est une illustration schématique d'un dispositif selon un mode de réalisation préféré de l'invention ;
- la figure 3 est un exemple d'étapes de traitement ou workflow connu selon le protocole Tuxedo.

### Définitions

Comme utilisé ici, les termes suivants ont les significations suivantes:
Le terme gène est l'unité fonctionnelle de l'hérédité. Chaque gène occupe une place spécifique (ou locus) sur un chromosome, est capable de se reproduire exactement à chaque division cellulaire, et de diriger la formation d'une protéine. Le gène comme une unité fonctionnelle peut être constitué d'un segment distinct d'une molécule d'ADN contenant le nombre approprié de purine :adénine (A) et la guanine (G) et pyrimidiques :cytosine (C), la thymine (T) des bases dans la séquence correcte pour coder la séquence d'acides aminés nécessaires pour former un peptide spécifique.

Le terme génome correspond à l'effectif total de gènes d'un ensemble de chromosomes trouvés dans des formes de vie supérieures, ou, les arrangements fonctionnellement similaires, mais plus simple, linéaire trouvés dans les bactéries et les virus. Un génome peut comprendre ou être représentée comme l'ADN génomique ou d'ADNc et peut également inclure l'ADN mitochondrial.

Une protéine est une macromolécule constituée de longues séquences d'alpha-amino-acides en liaison peptidique impliqué dans des structures, des hormones, des enzymes, et des fonctions essentielles de la vie.

Les termes profil d'expression génique décrivent généralement la synthèse des gènes étant exprimée en une seule fois dans une cellule. Des données d'informations génomique sur tout ou partie d'un génome.

Le terme pathologie signifie l'interprétation des maladies en termes d'activités cellulaires, à savoir, la manière dont les cellules deviennent instables.

Le terme mutation signifie un changement dans les caractéristiques d'un gène qui est commise dans les divisions ultérieures de la cellule dans laquelle elle se produit, ou un changement dans la séquence de paires de bases dans la molécule chromosomique.

L'ADN génomique forme la totalité de l'ADN chromosomique d'une forme de vie.

L'expression génique représente les gènes qui sont transcrits à partir de l'ADN et traduits en protéines, mais aussi en référence à un tissu particulier, le stade de développement ou leurs combinaisons.

Signature génique est un résumé de l'expression des gènes en une seule fois dans un profil, généralement utilisé en référence à la pathologie, mais également en référence au stade de développement de l'organisme, une réponse à des stimuli tels que les médicaments ou les facteurs environnementaux, la spécificité tissulaire, l'âge, et la progression de la maladie.

D'autres termes sont utilisés ici sont bien connus de l'homme de l'art et n'ont pas besoin d'être décrits en détail ici.

### Description détaillée

Sauf indication spécifique du contraire, des caractéristiques techniques décrites en détail pour un mode de réalisation donné peuvent être combinés aux caractéristiques techniques décrites dans le contexte d'autres modes de réalisation décrits à titre exemplaire et non limitatif.

Les termes « machine maître » et « moyens de traitement centralisés » sont utilisés comme synonymes. De même, les termes « machine esclave » et « moyens de traitement répartis » sont utilisés comme synonymes.

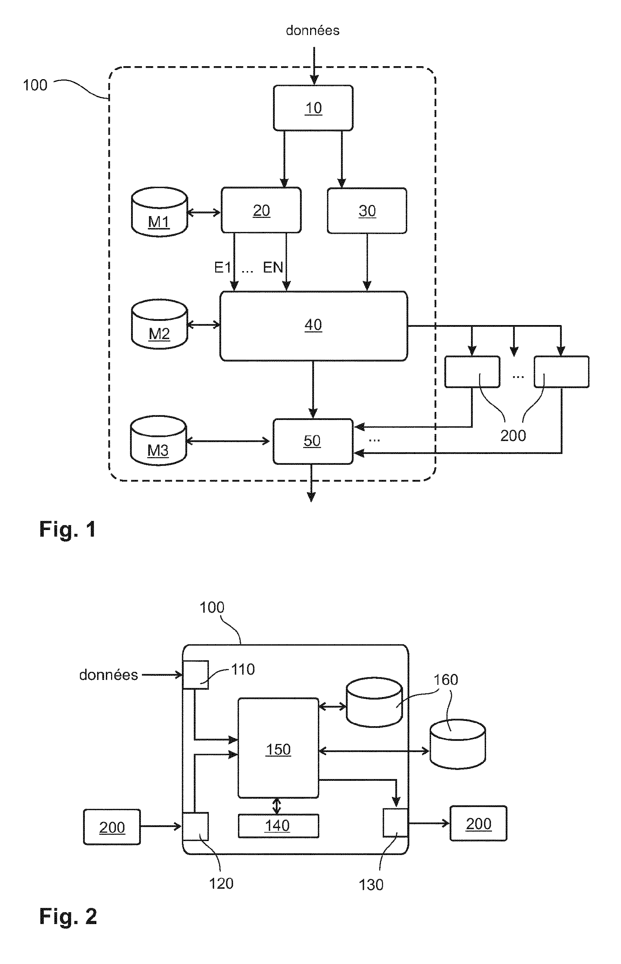

La figure 1 montre de manière schématique les étapes principales du procédé selon l'invention. A l'étape 10, les données d'entrée du procédé sont mises à disposition des moyens de traitement centralisés 100. Il s'agit de données génomiques ou de données transcriptomiques, ainsi que d'un type de traitement ou d'analyse à effectuer sur ces données. Les données peuvent être obtenues directement d'un dispositif séquenceur, ou elles peuvent être lues à partir d'une mémoire sur laquelle elles ont été enregistrées au préalable. Cette mémoire peut être un disque dur, une mémoire de type SSD, ou un server de données relié par un réseau de communication à la machine maître 100. Les moyens de lecture adéquats pour accéder à ces sources de données sont à la portée de l'homme du métier et ne seront pas décrites de manière détaillée dans le contexte de cette invention.

Lors de l'étape 20, les données d'entrée sont utilisées pour déterminer un workflow de traitement. Un workflow consiste en une séquence d'étapes (E1, ..., EN) de traitement de données qui doivent être réalisées selon un ordre précis afin de réaliser l'analyse requise des données. Dans un mode de réalisation préféré, l'ensemble des workflows disponible est enregistré sur un support de données M1 accessible par les moyens de traitement centralisés. En connaissant la nature des données fournies en entrée ainsi que le traitement global à appliquer, un ou plusieurs workflows peuvent ainsi être sélectionnés parmi un ensemble prédéterminé.

Lors de l'étape 30, les données d'entrée sont utilisées pour déterminer une valeur indicative de leur complexité. Cette valeur permet en outre de déterminer une allocation de ressources de calcul pour chacune des machines esclaves. Dans le cas précis des séquences d'ADN et/ou d'ARN, les fonctions de complexité sont bien connues de l'homme du métier. La mesure est calculée à l'aide d'une ou de plusieurs fonctions de complexité choisies parmi
(i) la fonction Wootton & Federhen, FCF ;
(ii) la fonction de l'entropie de complexité, CE ;
(iii) la valeur des modèles de Markov, CM ;
(iv) les valeurs linguistiques de complexité, CL ;
(v) la complexité Zlib, ZL ;
(vi) les valeurs Trifonov, CT ;
(vii) une normalisation de ces fonctions.

Lesdites fonctions de complexité sont généralement évaluées en calculant les paramètres suivants des données d'entrée:
- la taille d'une suite de nucléotides à identifier,
- la répétition d'une suite de nucléotides de taille prédéfinie,
- le ratio de la longueur totale du génome analysé pour chacun des nucléotides
- Teneur GC: Fonction pour calculer la teneur en GC
- Teneur AT: Fonction pour calculer la teneur en AT
- Teneur AT: fonction pour calculer la teneur en biais AT
- Teneur GCS: fonction pour calculer la teneur en biais GC (répétition des GC)
- Teneur CpG pour calculer la teneur en CpG (Cytosine phosphorylée Guanine)
- le logarithme d'une base quelconque
- la fonction de mots de comptage dans une séquence
- la fonction pour créer une séquence d'ADN aléatoire
- la fonction pour créer une liste de mots possibles avec une longueur spécifique

A titre d'exemple, la valeur de complexité peut être obtenue en évaluant la complexité selon Wootton & Federhen, et en additionnant le résultat aux valeurs entropiques de complexité, puis, en prenant le logarithme de base 2 de ce résultat.

En langage de script PERL, les fonctions correspondantes peuvent être implémentées comme suit :

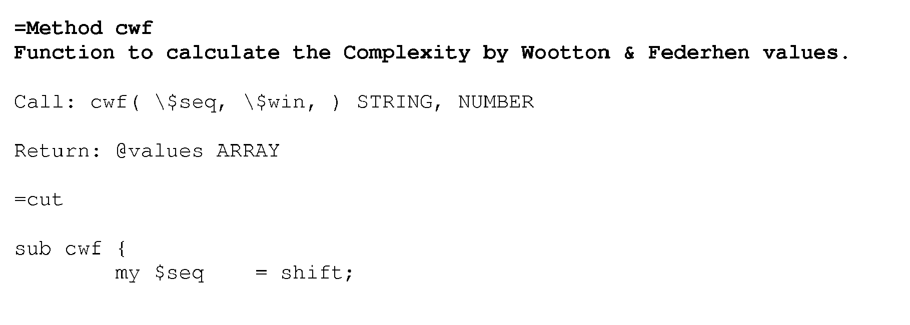

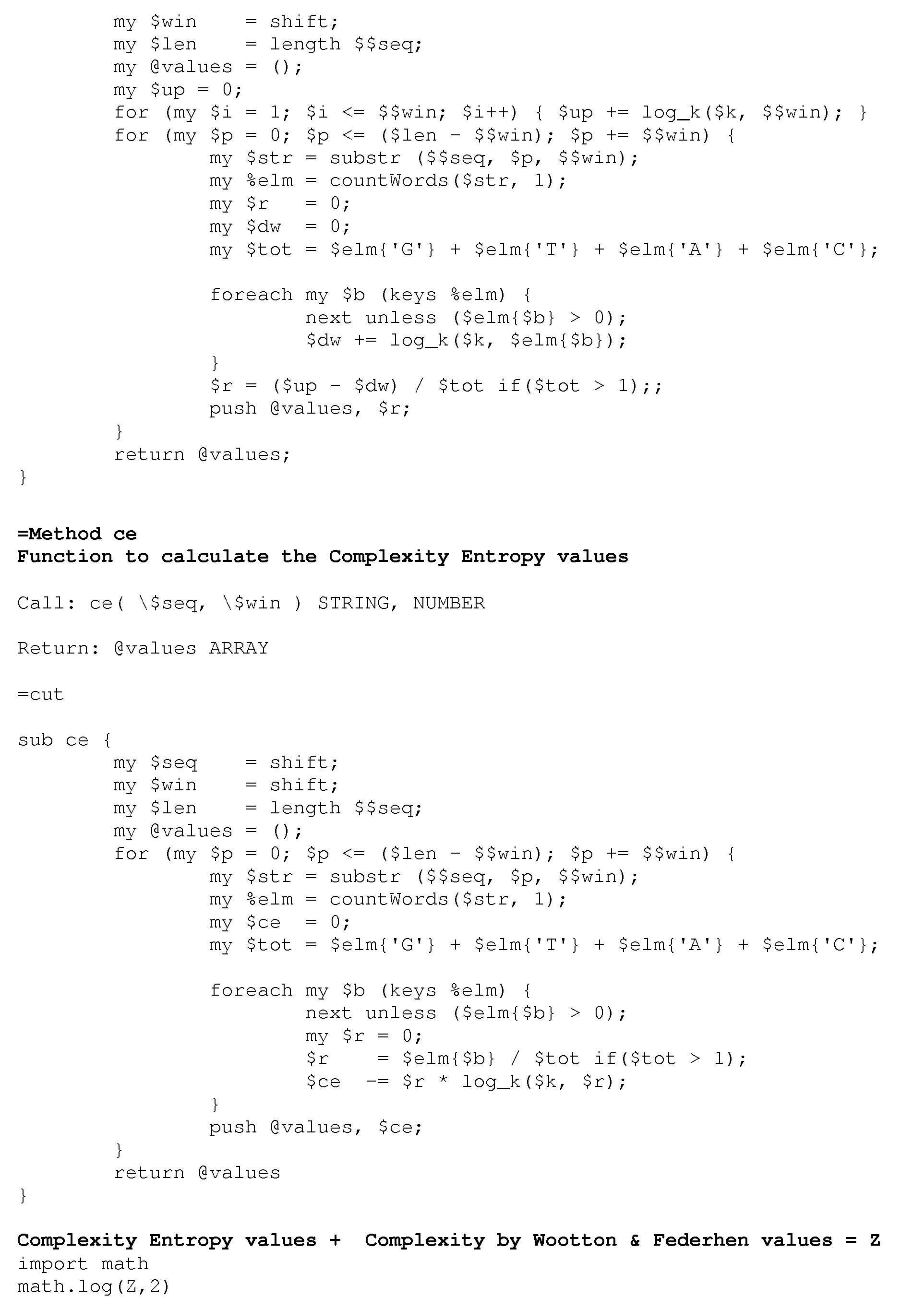

Si le résultat est inférieur 2.9, on peut par exemple quantifier la complexité de faible. Si le résultat se situe entre 2.9 et 3.3, on peut par exemple quantifier la complexité de moyenne. Si le résultat est supérieur à 3.3, on peut par exemple quantifier la complexité de haute. Il va de soi que cet exemple concret peut être adapté par l'homme de l'art selon l'application choisie et selon les moyens de calculs/de traitement disponibles.

Le résultat de l'étape 30 est donc une indication de la complexité des données d'entrées. A l'étape 40, ce résultat, ensemble avec les étapes du workflow déterminé par l'étape 20, servent à déterminer le nombre et la configuration des moyens de traitement répartis, qui sont destinés à réaliser lesdites étapes E1,..., EN. Pour reprendre l'exemple précédent, on peut considérer à titre non limitatif les ressources de calcul suivantes en rapport avec les niveaux de complexité faible, moyen et haut.
- Complexité faible : 1-2 CPU ; 1536MB RAM ; 1536MB mémoire SWAP ;
- Complexité moyenne : 4 CPU ; 6144MB RAM ; 6144MB mémoire SWAP ;
- Complexité haute : 4 CPU ; 8192MB RAM ; 8192MB mémoire SWAP.

Dans un mode de réalisation préféré, l'ensemble des ressources de calculs prédéterminés disponible est enregistré sur un support de données M2 accessible par les moyens de traitement centralisés.

Les données nécessitées pour réaliser les étapes E1,..., EN sont transmises aux machines esclaves ayant les configurations ainsi déterminées. De préférence, des machines virtuelles 200 ayant précisément ces configurations sont instanciées et les données d'entrées nécessaires au traitement leur sont transmises. La figure 1 montre par souci de clarté un exemple dans lequel les moyens de traitement répartis 200 réalisent leurs étapes de traitement respectives en parallèle. L'invention couvre également la situation selon laquelle une ou plusieurs étapes se suivent. Par exemple, la machine maître attend le résultat d'une première machine esclave, elle convertit le résultat vers un format adéquat selon le workflow déterminé, puis elle envoie les données converties vers une deuxième machine esclave pour effectuer une deuxième étape de traitement.

Une fois que chacun des moyens de traitement répartis à fini le traitement qui lui a été attribué, les résultats sont renvoyés au moyen de traitement centralisé lors de l'étape 50. Dans un mode de réalisation préférentiel soit les moyens de traitement répartis, soit les moyens de traitement centralisés, soit les deux types de moyens de traitement, enregistrent les données d'entrée, les configurations déterminées et les résultats obtenus dans une base de données M3. L'élément de stockage M3 contient donc tout le datapath utilisé pour obtenir les résultats d'analyse. Ces données peuvent être mis à disposition de tiers. Ceci permet la traçabilité et la reproductibilité du traitement de données ainsi effectué.

Le procédé selon l'invention permet est donc un procédé pour la structuration du flux de travail (workflow) et de la transmission de données nécessaires à l'analyse génomique mis en oeuvre par un ensemble de machines interconnectées comprenant au moins une machine maître et une ou plusieurs machines dites esclaves ou spécialisées pour exécuter une ou plusieurs analyses statistiques des données génomiques, remarquable en ce que la machine maître transmet le flux de données à une ou plusieurs desdites machines spécialisées ou esclaves pour exécuter ces analyses.

Les machines maîtres ou gestionnaires des flux de données génomiques et/ou transcriptomiques permettent non seulement d'automatiser les enchaînements de commandes diverses en fonction des niveaux de complexité prédéterminés, mais elles permettent aussi de garder la trace de tout le processus analytique et ainsi de faire face aux problèmes d'interopérabilité dus au chaînage de différents logiciels, et de les stocker pour leur réutilisation. En effet, l'application successive de divers programmes intervenant lors des étapes du workflow nécessite souvent de convertir les données produites par l'un des logiciels dans le format requis par l'autre, ce qui conduit la plupart de temps à effectuer des découper/coller et rechercher les expressions régulières dans des fichiers textes occasionnant une perte de temps et des sources d'erreurs et d'incohérence.

La figure 2 montre de manière schématique un dispositif selon un mode de réalisation préférentiel de l'invention. Le dispositif 100 implémente la fonctionnalité de la machine maître décrite par les étapes du procédé. Le dispositif comprend des moyens de lecture 110 adaptés à lire les données génomiques ou transcriptomiques d'entrée sur un support de mémoire. Le dispositif 100 comprend également des moyens de communication 120, 130 pour transmettre et recevoir des données vers/de des moyens de traitement répartis 200, un élément de mémoire 140 et des moyens de traitement 150. Les moyens de traitement 150 sont configurés de façon à réaliser les étapes décrites ci-dessous. Dans un mode de réalisation préférentiel, le dispositif a un accès en lecture et/ou en écriture sur un ou plusieurs éléments de stockage 160 soit locaux soit distants. De manière avantageuse, le dispositif 100 est un ordinateur. Des instructions ou moyens de codes lisibles par ordinateur sont avantageusement chargées dans l'élément de mémoire. L'exécution de ces instructions amène l'unité de traitement 150 à mettre en oeuvre le procédé selon l'invention.

A l'aide de la description fournie et des dessins accompagnant la description, l'homme du métier sait implémenter un programme d'ordinateur qui est apte à réaliser le procédé selon l'invention.

Ceci explique de manière générale le procédé selon l'invention. D'autres exemples de réalisation et des exemples d'étapes de traitement et de filtrage E1, ..., EN sont décrits dans ce qui suit. Il va de soi que ces exemples ne limitent d'aucune façon l'étendue de l'invention.

Les machines maîtres peuvent fonctionner avec des systèmes de gestionnaires des flux bien connus de l'homme du métier, tels que par exemple les systèmes Taverna, Knime et Galaxy. Taverna est un gestionnaire graphique codé en Java, développé par l'EBI (European Bioinformatics Institute). Il est orienté vers l'utilisation d'outils en bio-informatique sous forme de services web tels que NCBI, Kegg, Biomart, etc. Chaque étape E1, ..., EN des chaînes de traitements correspond à l'exécution d'un service disponible dans un menu en arbre. Parmi les fonctionnalités caractéristiques encore de Taverna, on note son comportement des constructions de contrôle telles que les conditions et les itérations, ce qui permet d'imbriquer des listes de données input en parallèle, en utilisant un certain nombre d'opérations prédéfinies. Il est doté également, d'un système de synchronisation automatique permettant l'interopérabilité des formats de données issues des diverses applications. Les workflows sont sauvegardés sous un format XML propre à Taverna : le SCUFL2.

Galaxy est une application web codée en Python spécifique développé conjointement par le Nekrutenko Lab (Center for Comparative Genomics and Bioinformatics, Penn State University) et le Taylor Lab (Emory University). Galaxy permet l'accès à une large panoplie d'outils bio-informatiques (SAMTOOLS, ClustalW, EMBOSS, Bowtie, etc.) massivement adaptés à la manipulation et à l'analyse des données génomiques. Il permet également d'accéder directement aux bases de données génomiques et plus particulièrement, aux bases de données UCSC et ENSEMBL.

Les machines maîtres et/ou esclaves sont de préférence hébergées dans le cloud afin de permettre les calculs informatiques et le stockage des données appropriées aux utilisateurs dans des environnements d'échange sécurisés.

Les machines maîtres et/ou esclaves sont connectées au serveur via un protocole Secure SHeI (SSH). L'authentification se fait grâce à la génération d'une paire de clés spécifiques à la machine (une clé privée et une clé publique). La clé publique peut être exportée sur chaque hôte sur lequel on souhaite pouvoir se connecter, tandis que la clé privée permet de prouver son identité au serveur. Ceci permet de garantir un accès à distance sur la console, en lignes de commande (shell) et d'effectuer toutes les opérations courantes et d'administration sur la machine distante. L'étape de construction du flux des données génomiques est effectuée par la machine maître ou gestionnaire du flux des données génomiques, depuis le début (sélection des données) jusqu'à la fin (affichage des résultats).

Ainsi, la machine maître agit en gestionnaire de flux et détermine l'exécution etl'enchainement d'un ensemble de tâches analytiques différentes, de manière automatique et traçable selon une fonction de complexité déterminée.

Selon la nature des données et leur niveau de complexité, la machine maître crée une machine virtuelle esclave qui est préconfigurée pour traiter le flux de données génomiques spécifique à chaque niveau de complexité. Le flux de données génomique est donc envoyé par la machine maître à une machine esclave ou spécialisée à laquelle est assigné un calcul statistique spécifique ou un travail d'analyse des données génomiques reçues. Ladite machine esclave est préconfigurée pour effectuer le flux de données approprié au niveau de complexité.

L'ensemble de ces différentes tâches analytiques prédéterminées par la machine maître sont réalisées par les différentes machines spécialisées ou esclaves et par un assemblage d'outils bio-informatiques. Cet assemblage se fait par l'intermédiaire de langage de programmation ou scripts écrits en PerI, shell, Python, etc...

La plupart des études bio-informatiques de la génomique font appel à une pluralité de logiciels afin de répondre à une question biologique donnée, ce qui induit généralement des problèmes de traçage du processus analytique et de reproductibilité, dus à des biais de paramétrage qui peuvent subvenir entre deux expériences identiques portant sur des données différentes. L'automatisation du processus analytique devient donc nécessaire et fait appel au concept de workflow ou de pipeline afin de produire des enchaînements de commandes diverses et de les stocker pour la réutilisation.

Selon un procédé préférentiel de la présente invention, la machine maître ou esclave peuvent fonctionner avec le système Galaxy en version web ou version locale ou encore installé sur un disque persistent. De manière plus préférentielle, la machine maître fonctionne sous système Galaxy installé localement.

De manière préférentielle, la présente invention utilise le procédé du cloud computing ou nuage informatique. Les machines maîtres et les machines esclaves sont donc hébergées dans le cloud ou nuage informatique. Les machines maîtres et esclaves sont donc organisées en des machines virtuelles et distantes permettant un accès à la fois facile et sécurisé aux différentes données des traitements génomiques, et l'exécution correcte des divers outils d'analyse des données NGS.

Le cloud computing est un concept qui consiste à déporter sur des serveurs distants, des stockages et des traitements informatiques traditionnellement localisés sur des serveurs locauxou sur des postes individuels. Selon le NIST, le cloud computing est l'accès via le réseau, à la demande et en libre-service, à des ressources informatiques mutualisées. La gestion de cette informatique délocalisée doit permettre donc aux utilisateurs du cloud de traiter leurs données dans des environnements informatiques sûrs et rapidement adaptables aux besoins en calcul et stockage. Les hébergeurs ou services cloud sont bien connus de l'homme du métier, et on peut citer par exemple Amazon, OVH, ou Gandi.

Dans un mode de réalisation de la présente invention, les d'outils d'analyse des données NGS sont installés et intégrés dans le cloud via la configuration de formats de fichiers NOSQL (Not Only SQL). L'administration de ces outils comprend ainsi la définition des icônes correspondants, l'organisation de l'ordre de leur mise à disposition en liste et enfin la définition des inputs, des outputs et des commandes d'exécution.

La présente invention a en outre pour objet un procédé d'analyse des données génomiques comprend au moins les étapes suivantes :
(i) une étape de détermination de la nature et filtrage des données génomiques brutes,
(ii) une étape de construction du flux des données génomiques brutes et traitées, et
(iii) une ou plusieurs étapes de connexion à des bases de données dites persistantes,
(iv) une étape de décision du traitement du flux de travail, et optionnellement
(v) une étape de stockage des résultats des données génomiques traitées, dans lequel une machine maître définit le traitement à appliquer au flux de travail en transmettant celui-ci à une ou plusieurs machines spécialisées ou esclaves. Les données génomiques qui sont traitées ou analysées selon la présente invention peuvent être obtenues par séquençage selon les nouvelles technologies de séquençage ou NGS. On peut citer à cet égard, les trois plateformes de séquençage NGS les plus populaires que sont Roche 454®, Illumina® et SOLiD®. Elles sont généralement divisées en deux catégories : lesplateformes produisant de larges quantités de lectures courtes (Ilumina® et SOLiD®) et les plateformes produisant des lectures plus longues en moindre quantité (454®). Il est à noter que chacune de ces plateformes est en constante évolution, ce qui entraîne une augmentation du débit, de la longueur et de la qualité des lectures de séquences.

Le système de pyroséquençage Roche/454® a été la première technologie de séquençage de nouvelle génération introduite sur le marché en 2005. Ce fut donc l'occasion d'appliquer pour la première fois les technologies NGS pour le re-séquençage du génome humain complet du Dr James Watson.

Illumina® est la seconde technologie NGS récente. Les brins d'ADN sont fragmentés aléatoirement par nébulisation et des adaptateurs sont fixés à chaque extrémité des fragments. Pour cette technologie, l'amplification des fragments se fait en phase solide. Les fragments sont fixés arbitrairement sur une surface par hybridation des adaptateurs. Les extrémités libres des fragments immobilisés s'hybrident à une amorce complémentaire (amplification par ponts) et sont copiés par la polymérase. Ce processus d'amplification est ensuite répété, aboutissant à la formation de clusters de brins d'ADN. Plusieurs millions (100 à 200) de clusters sont ainsi amplifiés simultanément sur la surface. En fin d'amplification, chaque cluster contient jusqu'à 1000 molécules identiques. Cette haute densité de brins d'ADN facilite et augmente le débit de séquençage. Le séquençage des fragments amplifiés se fait via un procédé de séquençage par synthèse qui incorpore les quatre nucléotides, marqués par des fluorochromes différents, au sein de brins d'ADN immobilisés. Comme pour la technique de Sanger, cette technique est basée sur l'utilisation de terminateurs réversibles contenant un groupement de protection attaché au nucléotide qui termine la synthèse d'ADN. A chaque cycle de séquençage, les quatre terminateurs réversibles ainsi que des amorces et la polymérase sont ajoutés sur la plaque de verre. Les terminateurs réversibles sont alors incorporés dans les brins d'ADN. Après l'excitation laser, l'image de la fluorescence émise par chaque cluster est capturée par lecture optique. Chaque cycle se termine par le clivage du terminateur et la restauration du groupement fonctionnel du nucléotide incorporé, ce qui permet à l'ADN polymérase d'incorporer le prochain nucléotide lors du cycle suivant. Chaque base possède une longueur d'onde spécifique d'excitation, ce qui permet au fur et à mesure des cycles répétés de déterminer la séquence de chacun des fragments, base par base. Cette plateforme permet de fournir jusqu'à 35Mb avec des lectures allant de 75pb à 100pb.

Avec la technologie ABI SOLiD Applied Biosystem®, l'amplification est réalisée par émulsion PCR sur des billes qui sont ensuite fixées sur une plaque de verre. Par la suite, le système SOLID® utilise une technique de séquençage par ligation. Ainsi la détection de la base ne se fait plus par l'intégration des nucléotides via la polymérase mais par l'incorporation de sondes par une ligase, chacune des sondes représentant un dinucléotide. Bien qu'il y ait 16 paires de bases possibles, ce système n'utilise que quatre couleurs pour les représenter. Ces couleurs codent les transitions entre deux nucléotides successifs. Chaque base est donc, codée deux fois par deux couleurs successives. Ce système permet une correction interne des erreurs lors de l'alignement. Les données de séquences sont codées sous la forme d'une succession de données de couleurs, appelée «colorspace». Cette technologie fournie jusqu'à 50Gb de lectures courtes allant de 25 à 75 pb.

Selon cet aspect de la présente invention, il est possible d'utiliser les technologies de séquençage dites de troisième génération qui sont encore plus récentes. Parmi celles-ci on reconnait Ion Torrent de Life technologies. Cette technique procède de la même façon au niveau des cycles d'amplification de l'ADN, mais utilise une méthode différente de séquençage qui se repose, essentiellement sur la mesure du largage des hydrogènes lors de l'incorporation des bases dans un brin d'ADN. Ceci permet un décryptage plus facile et réduit la durée du séquençage.

Alternativement, il est possible d'utiliser des technologies de séquençage encore plus avancées par nanofabrication et microscopie optique qui permettent le séquençage en temps réel, sans aucune amplification d'ADN préalable nécessaire. Ces nouvelles technologies garantissent un gain de temps considérable et surtout éliminent les biais d'erreurs liés aux PCR.

A l'issue du séquenceur, les données génomiques sont importées et sont traitées. L'importation de ces données génomiques peut se faire de plusieurs manières selon la source du fichier. De préférence, l'importation se fait à partir d'une URL ou directement partir d'une base telle que la base UCSC en utilisant un client FTP tel que par exemple Filezilla afin d'accélérer le processus.

Le traitement des données de séquençage se fait en trois stades d'analyses différents, qui peuvent donc être des étapes d'un workflow donné: les analyses primaires, les analyses secondaires et les analyses tertiaires. Ces différentes étapes visent à couvrir le traitement de données génomiques spécifiques à l'instrument de séquençage jusqu'aux analyses finales liées aux différentes interprétations biologiques (Data mining, annotation, etc..).
Dans un mode de réalisation de la présente invention, la machine maître détermine, selon le calcul de la fonction ou du niveau de complexité, la transmission des données nécessaires à l'analyse des données génomiques et crée les machines spécialisées ou esclaves qui ont pour tâche d'exécuter les analyses grâce à une liste prédéterminée d'outils dédiés aux analyses secondaires et d'outils d'analyses tertiaires des données NGS de manière simple et reproductible. Ces outils sont par exemple les logiciels ToPhat, Bwa, Bowtie, FastQc, etc...

Cette étape de traitement des données génomique est caractérisée en ce que la machine maître définie le traitement à appliquer au flux de travail en transmettant celui-ci a la machine spécialisée selon une fonction de complexité qui a été préalablement calculée comme décrit précédemment.

Dans un mode de réalisation de la présente invention, le traitement des données génomiques comprend une première étape de détermination de la nature des données génomiques. Les données génomiques disponibles pour un organisme étudié se présentent le plus souvent sous la forme de données ARN-Seq ou ADN séquencé, mais également sous d'autres formes telles que des séquences d'acides aminés, des voies métaboliques ou de signalisation.

Le traitement des données génomiques peut ensuite comprendre des analyses primaires à savoir des études statistiques qui sont menées sur les fragments de lectures « reads » afin d'évaluer leur fiabilité et la qualité des données. Des scores de qualité sont ainsi attribués à chaque base séquencée permettant d'estimer les taux d'erreurs du séquençage.

La deuxième étape concerne les analyses secondaires, au cours desquelles, des filtrages sont effectués sur les «reads » selon la qualité des séquences et en fonction des objectifs de l'étude menée. Les « reads » sont par la suite assemblés soit de novo soit alignés sur un génome de référence.

Cette étape de filtrage est recommandée car les taux d'erreurs et les biais systématiques de séquençage ont une influence sur la détection des informations telles que des Single Nucleotide Polyphormism (SNP). Il est en effet connu que les types d'erreurs sont différents selon les NGS et qu'il existe des biais qui leur sont intrinsèques, par exemple Illumina® produit un taux d'erreurs qui augmente sur la longueur du read, et il en produit davantage sur les nucléotides G et C.

L'étape de filtrage comprend le nettoyage des données NGS, l'alignement et assemblage, et le mapping des transcrits. Cette étape de filtrage ou de contrôle de qualité des données génomiques peut être effectuée par la machine maître ou une machine esclave spécialisée.

L'étape de nettoyage des données génomiques peut être réalisée en plus de l'étape de filtrage.

Cette étape peut être réalisée par différents outils, tels que notamment FASTQTrimmer. Ceux-ci consistent à fixer un seuil de qualité en dessous duquel les bases aux extrémités 3' ou 5' présentant une mauvaise qualité sont éliminées.

Les logiciels d'alignement et d'assemblage des lectures de séquençage NGS nécessitent souvent d'indexer les génomes de références, afin d'accélérer les processus de mapping. Il Dans cette optique, il est possible de télécharger le génome humain de référence complet hg19 http://www.ncbi.nlm.nih.gov/projects/genome/assembly/grc/human/ sous format Fasta à partir du site FTP de Complete Genomics ou Ensembl. Il est possible d'indexer le génome en utilisant un logiciel de mapping tel que par exemples les logiciels Bowtie et ToPhat.

L'étape suivante dans le processus d'analyse, consiste à détecter les variations génomiques et les différences nucléotidiques (SNPs, insertions, délétions, etc..).

Enfin, l'analyse tertiaire consiste à associer les données issues des étapes précédentes avec des données phénotypiques expérimentales afin d'en tirer de manière statistique, des conclusions biologiques significatives. Le choix de la stratégie et de la méthodologie de l'analyse bio-informatique est diffèrent selon la nature de ces données génomiques. Une large panoplie d'outils bio-informatique est disponible dans le domaine. On peut ainsi citer différentes catégories de logiciels bio-informatiques tels que, les outils statistiques d'analyse de la qualité des données de séquençage (FastQc), les programmes d'alignement et d'assemblage des lectures de séquençage (Bwa, Bowtie, ToPhat, etc...), ou les logiciels de visualisation des données génomiques (IGB, IGV, etc.). Enfin, la plupart des études bio-informatiques de la génomique font appel à une pluralité de logiciels afin de répondre à une question biologique donnée, ce qui induit généralement des problèmes de traçage du processus analytique et de reproductibilité, dus à des biais de paramétrage qui peuvent subvenir entre deux expériences identiques portant sur des données différentes. Ces problèmes de traçabilité sont résolus grâce à la virtualisation des différentes machines et du stockage de la construction du flux des données génomiques brutes et traitées. Du fait de cette virtualisation, le flux de travail et les résultats (conversion des données brutes et données traitées) peut être conservées.

Selon un mode de réalisation préférentiel de l'invention, le procédé peut être utilisé afin d'implémenter un traitement des données connu, consistant à analyser des schémas de niveaux d'expression d'au moins un gène dans au moins une expérience ou au moins une pathologie sont déterminés (Trapnell et al., Nature Protocols 7, 562-578, 01 March 2012). Ce protocole d'analyse différentielle constitue une étape clé, en vue de l'exploration des gènes différentiellement exprimés entre au moins deux conditions différentes, par exemple, deux tissus différents (tissus surrénaliens, tissus du cerveau humain, etc...), ou tissus issus de patients malades et sains, ou tissus issus de patients traités et non traités, ou encore tissus de patients traités avec différentes drogues.

Selon ce mode de réalisation préférentiel, l'étape d'analyse des données génomiques comprend par exemple un protocole d'analyse différentielle de l'expression des transcrits ARNseq dans différentes conditions, le mapping des transcrits, et la visualisation graphique des mappings ainsi obtenus. Le protocole d'analyse différentielle des transcrits faite par les machines esclaves spécialisées après le processus de nettoyage qui est effectué par la machine maître ou une machine esclave.

Plusieurs étapes sont nécessaires pour l'analyse différentielle de l'expression des transcrits correspondant aux différentes conditions testées. Dans un premier temps, les transcrits correspondants à chacun d'au moins deux types de tissus ou conditions sont alignés séparément, par exemple avec l'outil ToPhat, sur le génome de référence humain. Ces étiquettes d'ARNm mappées sont par la suite assemblées par exemple via l'outil Cufflinks, afin de générer pour chaque type de tissu un fichier contenant tous les transcrits assemblés et leurs gènes correspondants.

Les deux fichiers d'assemblage des transcrits sont fusionnés par exemple avec l'outil Cuffmerge, correspondant aux différentes conditions, avec l'annotation du génome de référence. L'ensemble des gènes, y compris ceux dont les niveaux d'expression sont assez faibles pour pouvoir être reconstruits, peut être visualisé dans un seul fichier avec une annotation finale. L'annotation finale peut ainsi comprendre les isoformes nouvellement détectés suite au mapping des transcrits sur le génome de référence.

Enfin, l'analyse différentielle de l'expression des gènes peut être effectuée, par exemple via l'outil Cuffdiff, qui permet de comparer statistiquement, les niveaux d'expression des gènes dans les différentes conditions, en tenant compte à chaque fois de l'abondance relative des étiquettes d'ARNm issues de l'étape précédente de mapping. Cette analyse des données génomique repose donc sur le principe que le niveau d'expression des gènes est proportionnel à l'abondance des reads ARNm correspondants.

La dernière étape du traitement du flux des données pour l'analyse différentielle des données génomiques consiste à utiliser l'outil Cuffdiff afin d'explorer les gènes différentiellement exprimés dans différentes conditions (par exemple des tissus surrénaliens et des tissus du cerveau). Cuffdiff effectue des tests statistiques de comparaison des niveaux d'expression des gènes entre les deux tissus, via un algorithme qui prend en entrée d'une part, le fichier d'annotation consensus (merged_ transcripts) issu de Cuffmerge et d'autre part les deux fichiers Accepted_hits générés préalablement par ToPhat et correspondants aux deux types de tissus. Plusieurs fichiers sont générés par Cuffdiff.

Selon un autre mode de réalisation préférentiel de la présente invention, l'étape de traitement des données génomiques permet de diagnostiquer une pathologie à partir des résultats de tests d'expression génique pour un groupe de test ayant la pathologie et des résultats de tests d'expression génique pour un groupe contrôle. Les résultats des tests d'expression génique entre les groupes test et contrôle sont analysées comparativement pour obtenir une liste de gènes candidats probables et leurs niveaux de confiance associés.

L'analyse peut être effectuée par le calcul d'une moyenne et d'un écart-type des résultats de test d'expression du gène pour un premier gène codant pour le premier groupe de test et pour le groupe de contrôle, le calcul d'une valeur t pour une hypothèse de ce que les moyennes pour le premier groupe et pour le groupe de contrôle sont égales, et la détermination d'une valeur de confiance pour la valeur de t qui est calculé. Si l'hypothèse selon laquelle les moyennes pour le premier groupe et le groupe de contrôle sont égales est rejetée, le premier gène est inséré comme un candidat potentiel pour la pathologie. Le calcul d'une moyenne et écart type, le calcul d'une valeur de t et la détermination d'une valeur de confiance sont réalisées les autres gènes dans le groupe test et le groupe témoin, de façon à obtenir une liste de gènes candidats probables et les niveaux de confiance associés.

Les données d'expression génique sont affichées par le tri des ensembles de données d'expression de gène pour au moins un essai sur la base d'une famille de gènes à laquelle appartient chaque gène. Les données d'expression génétique pour la pluralité des expériences sont affichées, regroupées par famille de gènes.

Le procédé selon la présente invention comprend une étape supplémentaire de stockage des données analysées. Dans cette étape de stockage, la machine maître définit l'allocation des ressources en base de données de manière dynamique. Les résultats d'analyse des données génomiques sont donc stockés dans une machine spécialisée de préférence virtuelle délocalisée dans le cloud et qui est chiffrée de façon à assurer une sécurité maximale aux données génomiques ainsi traitées.

Une sécurité maximale est assurée en utilisant de préférence un firewall (logiciel parefeu) (iptable) sur les machines pour les flux entrants et sortants. De manière encore plus préférentielle, les services tels que dns, web (http et https) sont autorisés par des connexions distantes (ssh), et les protocoles de copie (rsync, scp) passent par le port 22 (ssh). Tous les autres paquets seront rejetés.

Dans certains modes de réalisation, les données d'expression de gènes peuvent être triées par l'obtention des résultats expérimentaux et un changement minimal entre l'expression desgènes ou une autre structure de données.

Les données génomiques et l'identification de la source peuvent également être stockées dans une bibliothèque d'objets génomiques provenant d'une source externe. Une pluralité de modules de génération de l'objet est appliquée aux données génomiques. Un objet est ensuite stocké dans une bibliothèque orientée objet qui inclut une hiérarchie de classes parentes et sous-classes qui représentent des données génomiques objet. Par exemple, les données génomiques peuvent être obtenues à partir d'une base de données publique, tels que l'Institut national de la santé (NIH), base de données, qui est externe à la base de données orientée objet. Le module objet de génération peut être instancié sur la base de l'identification de la source, et le module de génération de l'objet sélectionné peut instancier au moins un objet. Les données génomiques sont ensuite analysées pour déterminer les variables connexes pour les données génomiques ainsi que les méthodes qui sont exécutées.

Dans certains modes de réalisation, le nouvel ensemble de données de génomique est obtenu en réponse à l'entrée d'utilisateur. Dans d'autres réalisations, le nouvel ensemble de données de génomique est obtenu en réponse à un robot de base de données qui analyse un réseau informatique. Les données génomiques peuvent être fournies de deux façons soit par l'utilisateur soit par unrobot qui les cherche sur un réseau tel que le www.

Une interface utilisateur est mise en place avec un système client qui est sensible à une entrée d'utilisateur pour générer les demandes d'analyse de données brutes avec un générateur d'objet et avec une pluralité d'outils d'analyse de données génomiques.

On comprendra que des modes de réalisation de l'invention peuvent être fournis sous forme de systèmes, méthodes, machines et/ou des produits de programmes informatiques.

### EXEMPLES

### Exemple 1 : Identification de bio-marqueur spécifique du poumon avec le protocole TUXEDO

Cet exemple illustre d'un exemple concret de conception d'un workdlow pour l'analyse de données RNA-Seq issues du projet : Human BodyMap 2.0 data from Illumina (numéro d'accession EMBL : E-MTAB-513). Un tel workflow peut être parmi les workflow prédéterminés utilisés lors de l'étape 20 selon le procédé de l'invention.

Il s'agit d'analyser l'expression différentielle des gènes dans 16 tissus humains différents (tissu surrénalien, tissu adipeux, du cerveau, du sein, du côlon, du coeur, des reins, du foie, du poumon, de la lymphe, de l'ovaire, de la prostate, le muscle squelettique, des testicules, de la thyroïde, et des globules blancs), et ainsi d'identifier des biomarqueurs de gènes spécifiques du cancer du poumon.

L'objectif de cet exemple est de montrer la construction de la transmission du flux des données génomiques en vue de l'analyse différentielle de l'expression des gènes entre les tissus surrénaliens et les tissus du cerveau humain. Les différentes étapes correspondent aux étapes E1,..., EN de la Figure 1, mises en oeuvre de manière automatique par le procédé selon l'invention.

### Exemple 1.1 Upload des données RNA-Seq

L'étude a porté sur des données RNA-Seq « paired-end » avec de lectures de 50pb, «reads» issues des tissus humains surrénaliens et du cerveau. Deux fichiers séparés en formats fastq (Annexe.3) pour chaque type de tissu sont crées correspondant aux lectures RNA-Seq sens et anti-sens, « Forward & Reverse reads » . Ces échantillons de données ont permis de couvrir en moyenne, une région de 500Kb du chromosome 19 humain et plus précisément, entre les positions 3-3.5 millions (chr19:3000000:3500000).

### Exemple 1.2 Contrôle de la qualité des reads

La qualité des reads a été analysée car les taux d'erreurs et les biais systématiques de séquençage ont une influence sur la détection des informations telles que les SNP. En effet, une étude récente sur la qualité des séquences a montré que les types d'erreurs sont différents selon les NGS et qu'il existe des biais qui leur sont intrinsèques. Par exemple Illumina® produit un taux d'erreurs qui augmente sur la longueur du read, et il en produit davantage sur les nucléotides G et C. Le processus de contrôle de qualité des reads a été effectué en plusieurs étapes, via les outils qui se répertorient sous le thème « NGS: QC and manipulation ».

### 1.2.1 - Fastq Groomer

Le logiciel FASTQ Groomer permet de convertir les différents formats des fichiers fastq. Il a été utilisé ici pour convertir les fichiers de reads vers le format fastq-sanger dont les scores phred qualifiant une bonne qualité de base, s'échelonnent de 33 à 73 (Annexe.3). En effet, les outils d'analyse statistique de la qualité des séquences prennent généralement en entrée des séquences au format fastq-sanger.

### 1.2.2 - FASTQ Summary Statistics

FASTQ Summary Statistics a été ensuite utilisé afin de générer à partir d'un fichier de données fastq-sanger, un tableau de chiffres statistiques liés à la distribution des scores phred, à travers les 50 paires de bases de chaque read séquencé. Le tableau ainsi obtenu comprend 50 lignes correspondant chacune à une base, dans une position donnée du read, en allant de l'extrémité 5'OH à l'extrémité 3'P. Parmi les informations importantes à tenir en considération dans ce tableau, valeur « med » qui correspond à la médiane des scores de qualité phred pour chaque position de base. Cette étape a ainsi permis d'obtenir les 2 tableaux statistiques de description de la qualité des reads (Forward et Reverse), pour chaque type de tissu.

### 1.2.3 - Visualisation de la qualité des reads

La qualité des reads a été évaluée grâce à la visualisation graphique de la distribution des scores phred le long des séquences grâce au logiciel FastQC. Des rapports HTML, comprenant des graphiques ont été générés afin de de repérer, de manière détaillée et simple les problèmes provenant des erreurs de séquençage. Un autre outil: Boxplot a été mis au point pour la visualisation graphique des scores de qualité.

L'analyse de la distribution graphique des scores phred via les deux logiciels montrait une qualité relativement satisfaisante pour l'ensemble des reads, avec parfois une diminution observée des valeurs des médianes, au niveau des bases situées aux extrémités 3'.

### 1.2.4 - Nettoyage des reads

Le nettoyage (trimming) des reads des bases de mauvaise qualité a été réalisé avec l'outil FASTQ Trimmer. Ceci consiste à fixer un score seuil de qualité en dessous duquel, les bases situées aux extrémités 3' ou 5' et présentant une mauvaise qualité seront éliminées. En l'occurrence, le seuil imposé était égal à 20. Étant donné que les reads présentaient dans la globalité de leurs bases des valeurs de médianes bien supérieures à 20, cette étape ne s'est pas révélée nécessaire.

### Exemple 1.3 Protocole d'analyse différentielle de l'expression des gènes

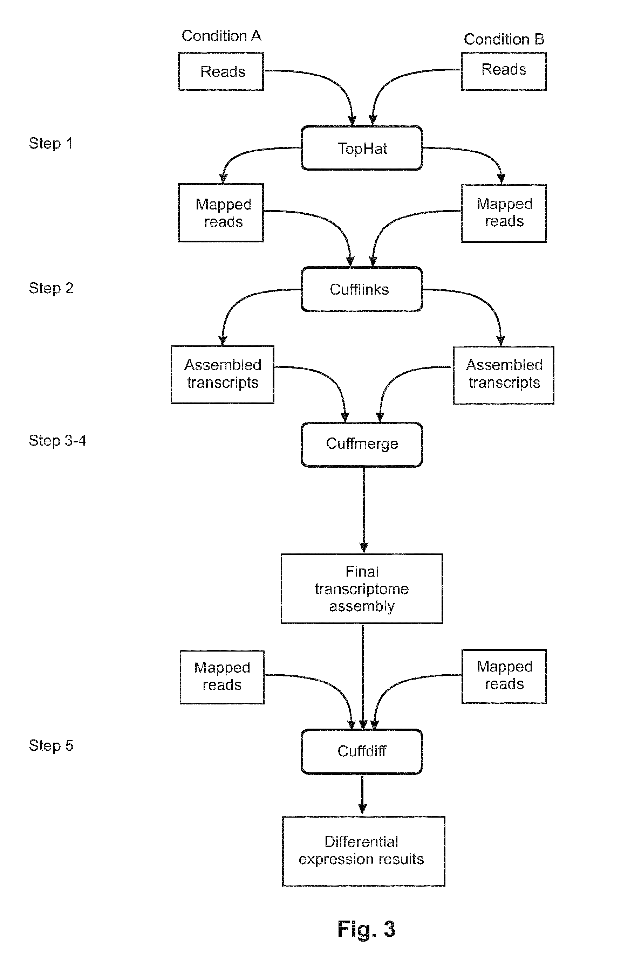

Ce protocole, illustré par la Figure 3, constitue une étape clé, en vue de l'exploration des gènes différentiellement exprimés entre les tissus surrénaliens et les tissus du cerveau humain, et a porté sur les données RNA-Seq sur lesquelles, le processus préalable de contrôle de qualité et de nettoyage des reads a été effectué. Plusieurs étapes étaient nécessaires pour l'analyse différentielle de l'expression des gènes.

Dans un premier temps, les reads correspondants à chacun des deux types de tissus, ont été alignés séparément, avec l'outil ToPhat [TM] sur le génome de référence humain. Ces étiquettes d'ARNm mappées, ont été par la suite assemblées via l'outil Cufflinks [TM], qui en produit pour chaque type de tissu, un fichier contenant tous les transcrits assemblés et leurs gènes correspondants. Ensuite, Cuffmerge a fusionné les deux fichiers d'assemblage des transcrits correspondants aux deux tissus, avec l'annotation du transcriptome de référence, le tout dans un seul fichier qui correspond à une annotation finale plus exhaustive et tenant compte de l'ensemble des gènes, y compris ceux dont les niveaux d'expression sont assez faibles pour pouvoir être reconstruits. Cette annotation finale comprendra aussi, les isoformes nouvellement détectés suite au mapping des reads sur le génome humain. Enfin, l'analyse différentielle de l'expression des gènes proprement dite, a été effectuée via l'outil Cuffdiff [TM] qui permet de comparer statistiquement, les niveaux d'expression des gènes dans les deux tissus, en tenant compte à chaque fois de l'abondance relative des étiquettes d'ARNm issues précédemment, du mapping ToPhat. Cette étude repose donc sur le principe que le niveau d'expression des gènes est proportionnel à l'abondance des reads ARNm correspondants.

### 1.3.1- Mapping des reads RNA-Seq

Le mapping des reads via ToPhat. Cette étape consiste à aligner les fragments de lectures d'ARNm sur le génome de référence humain. Cependant, un des problèmes d'alignement, spécifique des séquences transcriptomiques, provient du fait que certaines lectures s'étendent à travers les jonctions d'épissage à l'extrémité des exons et ne peuvent pas donc, être alignées directement sur la séquence génomique. Cette difficulté sera levée par l'utilisation de l'outil ToPhat; un logiciel spécifique pour le mapping des données RNA-Seq et la détection des jonctions d'épissage.

Les lectures RNA-Seq sont obtenues à partir des ARNm. Les programmes d'alignement, tels que Bowtie et Maq permettront d'aligner les lectures grises au génome de référence, mais échoueront à aligner les lectures traversant les jonctions exoniques. Des programmes, tels que ToPhat, peuvent permettre d'aligner ces lectures au génome de référence.

Etant donné que les données RNA-Seq dont on dispose, s'étendent sur une région de 500Kb du chromosome 19 humain, et afin d'accélérer le processus du mapping via ToPhat, nous allons aligner les reads (sens et anti-sens) sur le chromosome 19 et non pas sur tout le génome humain. Pour cela, il faut d'abord, importer le fichier fasta du chromosome 19 à partir de la librairie. A l'issue de cette étape, ToPhat génère 4 fichiers différents pour chaque type de tissu :
- Accepted_hits: est un fichier binaire d'extension Bam. Il comprend les différentes positions des fragments de reads alignés avec succès sur le génome de référence.
- Splice_junctions: Il s'agit d'un fichier d'extension Bed, qui reporte les positions des sites d'épissage couvrant les reads alignés.
- Insertions et deletions : Ces deux fichiers d'extension Bed indiquent les pistes d'insertions et de délétions reportées par ToPhat.
- La visualisation graphique du mapping.

La visualisation graphique du mapping des reads RNA-Seq sur le chromosome 19, s'effectue via deux outils. Il s'agit des deux logiciels graphiques : Trackster et IGV. Ces deux logiciels prennent en entrée, les fichiers output de ToPhat (Accepted _hits.bam et Splice_junctions.bed) et permettent donc la visualisation des fragments de reads alignés sur le génome humain et s'étendant à travers les jonctions d'épissage.

Toutefois, il est à noter que l'utilisation d'IGV nécessite d'abord, la compression du fichier bam, pour que le logiciel puisse le lire. Ceci s'effectue en convertissant le format bam en format bai.

### Assemblage des reads via Cufflinks

Après le mapping des reads, l'étape suivante consiste à assembler ces bouts de fragments d'ARNm générés par ToPhat, en transcrits complets. Ceci nous aidera à mieux observer les phénomènes de splicing des ARNm et surtout, nous mènera par la suite, à l'analyse différentielle de l'expression des gènes. Nous allons procéder donc, à l'utilisation du logiciel Cufflinks, qui permet de reconstruire de novo les transcrits à partir des bouts d'ARNm, issus précédemment du mapping ToPhat.

Toutefois, les résultats de l'assemblage dépendent de la profondeur de couverture des reads, et surtout de la prise en compte ou non d'un génome de référence dans les paramètres de Cufflinks. Ainsi, en absence de tout génome de référence lors de l'assemblage via Cufflinks, certains transcrits faiblement exprimés peuvent être incomplets. Cufflinks prend en entrée les données du mapping générées précédemment par ToPhat et plus précisément, le fichier Accepted_hits.bam. Il en génère pour chaque type de tissu, trois fichiers distincts :
- gene expression : Il s'agit d'un tableau qui donne une estimation des niveaux d'expression des gènes en FPKM.
- transcript expression: Il s'agit d'un deuxième tableau qui donne une estimation des niveaux d'abondance des transcrits en FPKM.
- assembled transcripts : Nous tiendrons compte de ce fichier pour la suite du protocole. Il s'agit d'un fichier en format gtf, reportant les coordonnés de l'ensemble des transcrits complets, avec les scores relatifs à leurs abondances. La visualisation graphique de l'assemblage des reads

Etant donné qu'on dispose cette fois ci des transcrits assemblés, la visualisation graphique des phénomènes de splicing d'ARNm sera plus claire. Nous allons procéder à travers cette visualisation graphique, à la comparaison de la qualité d'assemblage des reads via Cufflinks paramétré avec et sans une annotation génomique. Il est à noter que le fichier d'annotation de référence du chromosome 19, est sous format gtf, et sera importé à partir de la base de données UCSC. La qualité de l'assemblage des reads via Cufflinks, qui tient compte dans ses paramètres de l'annotation du génome de référence semble être beaucoup plus satisfaisante, par apport à celle effectuée en absence de toute annotation. On observe ainsi, un alignement concordant entre les transcrits assemblés et ceux de référence. De même, on observe plus clairement, les transcrits qui s'étendent à travers les jonctions d'épissage.

### Cuffmerge : Construction d'une annotation consensus de référence

L'assemblage des reads mappés contre le génome du référence permet de reconstruire des gènes complets ou incomplets selon les niveaux d'abondance des transcrits. De nouveaux isoformes de pseudogènes peuvent être également décelées. Par ailleurs, l'analyse différentielle de l'expression des gènes nécessite une annotation exhaustive qui tient compte de l'ensemble des gènes exprimés dans les deux tissus. Dans cette optique nous procédons à la construction d'une annotation consensus complète de référence, via l'outil Cuffmerge. Ce logiciel permet de fusionner dans un seul fichier, les données d'assemblage de transcrits générés par Cufflinks et qui correspondent aux deux types de tissus, avec l'annotation du transcriptome de référence (chr19).

Le fichier merged transcripts généré par Cuffmerge comporte donc, une annotation finale plus exhaustive, qui tient compte de l'ensemble des gènes, y compris les nouveaux isoformes et les gènes dont les niveaux d'expression sont très faibles.

### Cufflinks : Analyse différentielle de l'expression des gènes

La dernière étape du workflow consiste à utiliser l'outil Cuffdiff afin d'explorer les gènes différentiellement exprimés entre les tissus surrénaliens et les tissus du cerveau. Cuffdiff effectue des tests statistiques de comparaison des niveaux d'expression des gènes entre les deux tissus, via un algorithme qui prend en entrée d'une part, le fichier d'annotation consensus (merged_ transcripts) issue de Cuffmerge et d'autre part les deux fichiers Accepted_hits générés préalablement, par ToPhat et correspondants aux deux types de tissus. Plusieurs fichiers sont générés par Cuffdiff. Parmi ceux-ci, on note le fichier transcript differential_expression_testing.

Le fichier transcript differential_expression_testing représente un tableau statistique de comparaison des niveaux d'expression des gènes entre les tissus surrénaliens et ceux du cerveau. Les gènes différentiellement exprimés auront comme attributs de faibles p-value et « yes » dans la colonne « significant ». Ainsi, cette étude nous montre que parmi les gènes situés entre les positions 3-3.5 millions du chromosome 19, il existe un seul gène qui est différentiellement exprimé entre les deux tissus. Il s'agit du gène NM_198969 qui est surexprimé dans les tissus du cerveau et totalement absent dans les tissus surrénaliens. Le gène NM_198969 code la protéine AES (aminoterminal enhancer of split).

D'après les études de [Xin &, al, 2001], la protéine AES interagit comme un modulateur négatif de l'activité transcriptionelle des gènes impliqués dans l'inhibition de la différentiation tissulaire de la prostate chez l'homme. On peut donc à partir de notre étude, faire une première hypothèse sur l'implication de la protéine AES dans l'induction de de l'état de différentiation des tissus du cerveau.

## Revendications

1. Procédé de traitement informatique réparti de données génomiques ou transcriptomiques, comportant les étapes suivantes réalisées à l'aide de moyens de traitement centralisés:
- mise à disposition de données génomiques destinées à être traitées à l'aide de moyens de lecture faisant partie des moyens de traitement centralisés (10);
- détermination d'étapes de traitement nécessaires pour réaliser ledit traitement (20);
- calcul d'une mesure indicative du niveau de complexité des données génomiques (30);
- détermination de moyens de traitement répartis et de ressources de calcul allouées aux moyens de traitement répartis, en dépendance des étapes de traitement déterminées et de la mesure de complexité calculée, chacun des moyens de traitement répartis étant destiné à réaliser au moins une des étapes de traitement ;
- transmission d'au moins une partie des données génomiques et des ressources de calculs allouées à chacun des moyens de traitement répartis (40);
- réception des résultats de traitement générés par les moyens de traitement répartis (50).

2. Procédé selon la revendication 1, dans lequel les étapes de traitement sont sélectionnées par les moyens de traitement centralisés en fonction du traitement à réaliser parmi un ensemble d'étapes de traitement prédéterminé, stocké dans une mémoire accessible aux moyens de traitement centralisés.

3. Procédé selon une des revendications 1 ou 2, dans lequel les ressources de calcul allouées aux moyens de traitement répartis sont sélectionnées par les moyens de traitement centralisés parmi un ensemble de ressources de calcul prédéterminé, stocké dans une mémoire accessible aux moyens de traitement centralisés.

4. Procédé selon l'une quelconque des revendications 1 à 3, dans lequel les ressources de calcul allouées à chacun des moyens de traitement répartis comprennent une quantité de mémoire, une quantité de cycles de calcul ou une quantité de largeur de bande de disque pour être utilisée pendant la réalisation de l'étape de traitement associée aux moyens de traitement répartis.

5. Procédé selon une des revendications 1 à 4, dans lequel la mesure indicative du niveau de complexité des données génomiques ou transcriptomiques est calculée à l'aide d'une ou de plusieurs fonctions de complexité choisies parmi
(i) la fonction Wootton & Federhen, FCF ;
(ii) la fonction de l'entropie de complexité, CE ;
(iii) la valeur des modèles de Markov, CM ;
(iv) les valeurs linguistiques de complexité, CL ;
(v) la complexité Zlib, ZL ;
(vi) les valeurs Trifonov, CT ;
(vii) une normalisation de ces fonctions.

6. Procédé selon une des revendications 1 à 5, dans lequel le procédé comprend en outre une étape de stockage des données génomiques, des étapes de traitement déterminées, des ressources de calcul allouées aux moyens de traitement répartis réalisant les étapes de traitement, ainsi que des résultats générés par lesdits moyens de traitement distribués, de façon à enregistrer tout le datapath et de permettre la reproductibilité de toutes les étapes de traitement de données effectuées.

7. Procédé selon la revendication 6, dans lequel les données stockées sont écrites par les moyens de traitement centralisés dans une base de données à laquelle les moyens de traitement peuvent accéder.

8. Procédé selon une des revendication 1 à 7, dans lequel chacun des moyens de traitement répartis est configuré de façon à stocker dans un élément de mémoire les données génomiques reçues, l'allocation de ressources de calcul reçue et les étapes de traitement allouées par les moyens de traitement, ainsi que les résultats des étapes de traitement générés, de façon à permettre la reproductibilité de toutes les étapes de traitement de données effectuées.

9. Procédé selon l'une quelconque des revendications 1 à 8, dans lequel lesdits moyens de traitement répartis sont des moyens de traitement d'un dispositif informatique unique.

10. Procédé selon l'une des revendications 1 à 8, dans lequel lesdits moyens de traitement répartis sont des moyens de traitement de différents noeuds dans un réseau de communication.

11. Procédé selon l'une des revendications 1 à 10, dans lequel l'étape de transmission est réalisée à l'aide de moyens de transmission de données sécurisés.

12. Un programme d'ordinateur comprenant des moyens de code lisibles par ordinateur, qui, lorsqu'il est exécuté sur un ordinateur, amène l'ordinateur à exécuter le procédé selon l'une quelconque des revendications 1 à 11.

13. Un produit de programme informatique comprenant un support lisible par ordinateur sur lequel le programme d'ordinateur selon la revendication 12 est stocké.

14. Un ordinateur capable de mettre en oeuvre le procédé selon l'une quelconque des revendications 1 à 11.

15. Dispositif (100) pour la mise en oeuvre du procédé selon l'une quelconque des revendications 1 à 11, comprenant des moyens de lecture (110), des moyens de communication pour transmettre (130) et recevoir (120) des données vers des/de moyens de traitement répartis (200), un élément de mémoire (140) et des moyens de traitement (150) configurés de façon à :
- lire des données génomiques ou transcriptomiques destinées à être traitées à l'aide des moyens de lecture (110);
- déterminer un ensemble d'étapes de traitement nécessaires pour réaliser ledit traitement;
- calculer une mesure indicative du niveau de complexité des données génomiques;
- déterminer un ensemble de moyens de traitement répartis et de ressources de calcul allouées aux moyens de traitement répartis, en dépendance des étapes de traitement déterminées et de la mesure de complexité calculée, chacun de moyens de traitement répartis étant destiné à réaliser au moins une des étapes de traitement ;
- transmettre au moins une partie des données génomiques et des ressources de calculs allouées à chacun des moyens de traitement répartis;
- recevoir des résultats de traitement générés par les moyens de traitement répartis.
